# EUROPEAN PATENT SPECIFICATION

(11) **EP 4 743 033 B1**
(45) Date of publication and mention of the grant of the patent: **14.10.2026**
(21) Application number: 24762377.0
(22) Date of filing: 09.08.2024
(51) Int. Cl.: A61F 5/455, A61F 13/551

(54) **MENSTRUAL HYGIENE DEVICE**
MENSTRUATIONSHYGIENEGERÄT
DISPOSITIF D'HYGIÈNE MENSTRUELLE

(30) Priority: 16.08.2023 GB 202312483
(43) Date of publication of application: 20.05.2026
(73) Proprietor: LU Innovations Ltd, Glasgow G74 5BA (GB)
(72) Inventor: REID, Erin Kelly, Glasgow G74 5BA (GB)
(74) Representative: Specht, Jan Peter
(86) International application number: PCT/GB2024/052117
(87) International publication number: WO 2025/037094

(56) References cited:
- WO-A1-2022/114469
- KR-B1- 102 277 994
- US-A1- 2022 031 898
- US-B2- 11 369 509

## Description

### Field

The present disclosure concerns a menstrual hygiene device, a method of folding a menstrual cup for insertion into a vagina using the menstrual hygiene device and a kit of parts for use in the above method comprising the menstrual hygienic device and a menstrual cup.

### Background

WO 2022/114469 A1 discloses a menstrual cup storage container comprising a container part and a cover for opening and closing this container part. The well comprises a pressure tool with an insertion hole in the shape of a hollow channel for receiving a handle of a menstrual cup therein. When the cover part presses the cup downward towards the container part, the cup is simultaneously pressed by the cover part and the pressure tool and is folded multiple times as a result.

Periods are a part of the menstrual cycle where blood is released from the vagina for a few days. Sanitary products, such as sanitary pads, tampons or menstrual cups, can be used to absorb or collect the blood released during a period. Menstrual cups, which are growing in popularity due to their reusability, are typically made from silicone and are inserted into the vagina to collect the blood released during a period. In order to safely use sanitary products such as menstrual cups, the sanitary products must be clean and hygienically stored prior to use and hygienically handled during insertion into and removal from the vagina. However, when out in remote locations, for example when camping, hiking, orienteering etc., access to a suitable hygienic environment may not be possible and there may be limited access to bathrooms, hand washing facilities or sanitary bins. As such, there is a need for storing, applying, cleaning and/or disposing of menstrual products in a safe, hygienic way without the need for a hygienic environment, or bathroom or hand washing facilities.

### Summary of invention

The present invention solves this problem with a device according to claim 1, a method according to claim 10 and a kit of parts according to claim 15. According to a first aspect, there is provided a menstrual hygiene device comprising a housing and a well within the housing, the well comprising a post against which a menstrual cup is foldable using only the thumb and a finger of the same hand. As such, the menstrual hygiene device is suitable for folding a menstrual cup for insertion into a vagina. The menstrual hygiene device may also serve to store the menstrual cup before and/or after use by the user.

The menstrual hygiene device may be hand-held. The menstrual hygiene device may be portable. The housing of the menstrual hygiene device may take any suitable shape. The housing of the menstrual hygiene device may be elongate. The housing may be substantially prismatic. The housing may be substantially tubular (i.e. , tube-shaped). The housing may be substantially cylindrical (i.e., substantially circular in cross section perpendicular to a longitudinal axis). The housing may be substantially cuboidal. For example, the housing may be substantially square in cross section perpendicular to the longitudinal axis or the housing may be substantially rectangular in cross section perpendicular to the longitudinal axis. The housing may be triangular in cross section perpendicular to the longitudinal axis. The housing may be hexagonal in cross section perpendicular to the longitudinal axis.

The menstrual hygiene device may have a length of about 50 mm to about 250 mm, for example, from about 75 mm to about 225 mm, or from about 80 mm to about 200 mm. The menstrual hygiene device may have a length of about 50 mm to about 150 mm, for example from about 75 mm to about 125 mm, or from about 80 mm to about 100 mm. The menstrual hygiene device may have a length of about 100 mm to about 250 mm, for example, from about 125 mm to about 225 mm, or from about 150 mm to about 180 mm. The length of the device refers dimension along the longest longitudinal axis of the body of the device. The menstrual hygiene device may have a width of about 20 to about 150 mm, for example, from about 30 mm to about 100 mm, or from about 40 mm to about 80 mm, or from about 55 mm to about 75 mm. The width of the device refers to the dimension along the longest axis orthogonal to the longitudinal axis of the body of the device. The width may also be referred to as the diameter. For generally cylindrical shaped devices, the width will correspond to the diameter of the device.

The housing may be made from any suitable material. The housing may be made from a rigid material. The material may be a polymeric material. The material may be a plastic material. For example, the material may be a polyethylene, such as high-density polyethylene; polypropylene; or a styrene copolymer, such as acrylonitrile butadiene styrene. The material may be high-density polyethylene (HDPE). The material may be acrylonitrile butadiene styrene (ABS). The housing, or any part thereof, may be made by any suitable manufacturing process. For example, the housing may be made by moulding, such as blow moulding or injection moulding, preferably injection moulding. The housing may be manufactured by 3D printing. The housing may be provided with means (e.g., a connector) for attaching the device to a garment, bag or the like by known attachment means, for example a hook, a carabiner, or a rope. For example, the housing may comprise a ridge with an aperture on the housing, configured to enable attachment of the device to a garment, bag or the like by known attachment means, for example a hook, a carabiner, or a rope.

The post, which may also be referred to herein as a folding post, enables a user to fold the menstrual cup by pressing the menstrual cup against the post. For the avoidance of doubt, the post itself does not fold.

The post may be made from any suitable material. The post may be made from a rigid material. The post is solid, i.e. , is not hollow or does not comprise a channel or is not tubular. The post may be made from the same material as the housing. The post may be integrally formed with the housing.

The post may be positioned centrally within the well.

The post may extend away from a base of the well. For example, the post may extend substantially orthogonal to the base of the well. For example, a longitudinal axis of the post may extend substantially orthogonal to the base of the well. For example, the post (e.g., the longitudinal axis of the post) may extend away from the base of the well such that a smallest angle between the post (e.g., the longitudinal axis of the post) and the base of the well is no less than about 70°, for example, no less than about 80°, or no less than about 85°. The post (e.g., the longitudinal axis of the post) may be inclined relative to a normal to the base of the well by no more than about 20°, for example, no more than about 10°, or no more than about 5°.

The post may extend between a first end at a point of attachment to the base of the well and a second end distal from the point of attachment.

The post is typically elongate and may have any suitable cross-sectional shape. For example, the post may be generally slat shaped. The post may be generally column shaped. The post may be generally pillar shaped. The post may be cuboidal shaped. The post may be cylindrical shaped. The post may be triangular prism shaped. The side walls of the post may be substantially parallel to one another and orthogonal to the base of the well. One or both of the side walls of the post may be sloping. For example, one or both of the side walls may be sloping such that the side wall meets the base of the well at an acute angle (an angle less than 90°) or one or both of the side walls may be sloping such that the side wall meets the base of the well at an obtuse angle (an angle greater than 90°, but less than 180°). One or both of the side walls of the posts may slope from the first end of the post to the second end of the post, e.g., one of the side walls of the posts may slope from the first end of the post to the second end of the post. One or both side of the side wall of the post may comprise a parallel section and a sloping section across their length. The length of the post refers to the dimension along the longest longitudinal axis of the post. For example, one or both of the side wall have a sloping section from the first end of the post and a parallel section from the second end of the post. The sloping section may be half of the length of the post, e.g., from the second end of the post. The sloping section may be a third of the length of the post, e.g., from the second end of the post. The sloping section may be two thirds of the length of the post e.g., from the second end of the post.

The post may have a length of from about 10 mm to about 60 mm, for example from about 20 mm to about 50 mm. The post may have a length of from about 20 mm to about 50 mm, for example from about 30 mm to about 40 mm. The post may have a length of about 30 mm to about 60 mm, for example from about 40 mm to about 50 mm. The length of the post refers to the longest longitudinal axis of the post extending orthogonally from the base of the well to the distal end of the post. The post may have a width of from about 5 mm to about 40 mm, for example from about 10 mm to about 30 mm, or from about 10 mm to about 20mm, or from about 10 mm to about 15 mm. The width of the post refers to the longest axis of the post, orthogonal to the longest longitudinal axis, and disregarding any stop portion or section. The post may have a depth of from about 1mm to about 15 mm, for example, from about 2.5 mm to about 10 mm, or from about 4 mm to about 8 mm. The depth of the post refers to the longest axis of the post that is orthogonal to the length and width of the post.

The post may have a stop for retaining the menstrual cup in position against the post during folding. The stop may inhibit or prevent slippage of the menstrual cup as it is pressed against the post for folding. It may be that the stop is formed by a cut-out portion of the post. For example, the stop may be formed by a 90° cut-out portion.

It may be that the stop comprises a ledge. It may be that the ledge extends from the body of the post. The ledge may extend substantially orthogonally from the post. It may be that the ledge extends around the circumference of the post. The ledge may extend orthogonally around the circumference of the post. It may be that the ledge extends orthogonally around the circumference of the post, proximate the second end of the post.

It may be that the ledge is formed by a cut-out portion of the post, for example a 90° cutout portion of the post. It may be that the ledge is formed by a cut-out of the corner of the post, which provides a 90° cut-out portion of the post.

It may be that the stop (e.g., the ledge) is formed by a protrusion. The protrusion may be located on one side of the post or on two or more sides of post. The protrusion may extend around the entire post. The protrusion may extend around the circumference of the post. The protrusion may be located proximate the second end of the post.

It may be that the stop is formed by a notch of the post.

The stop may be positioned closer to the second end of the post than the first end of the post. The stop may be positioned proximate the second end of the post. The stop may be positioned up to about 10 mm from the second end of the post. The stop may be positioned at the second end of the post.

The stop may be shaped such that a corner of the second end of the post has an L-shaped profile (e.g., in cross-section). The stop may be shaped such that a corner of the second end of the post has a 90° cut-out.

The well may have a side wall. The side wall may extend around the well. Thus, the side wall may define the boundaries of the well. The side wall may have a height measured in a direction substantially orthogonal to the base of the well. The post may have a length greater than the height of the side wall. Therefore, the post may protrude out of the well beyond the side wall, i.e., when viewed from the side of the device. It may be that the stop is positioned beyond the side wall (i.e., outside the well). Alternatively, the post may have a length equal to or less than the height of the side wall. It may be that the stop is positioned on the post such that it is within the boundary of the well. The side wall of the well may have a height of from about 10 mm to about 50 mm, for example from about 15 to about 45 mm, or from about 20 mm to about 40 mm. The side wall of the well may have a height of from about 15 mm to about 35 mm, for example from about 20 mm to about 30 mm. The side wall of the well may have a height of from about 25 mm to about 45 mm, for example from about 30 mm to about 40 mm.

The housing may comprise a first section and a second section which are connectable to each other. The well may be comprised within (i.e., located within) the first section. When connected, the first and second sections may form a first chamber. It will be appreciated that a "chamber" refers to an enclosed space or cavity. It will further be appreciated that the first and second sections are independent components of the housing that said first and second sections being "connectable" refers to the fact that these sections may be connected (e.g., attached) to one another and may be disconnected (e.g., separated or released) from one another. The first and second sections may be connectable by (e.g., held together in a connected configuration by) any suitable fastening. For example, the first and second sections may be connectable by a screw (i.e., threaded) fastening, a latch fastening, a snap-fit fastening, or a magnetic snap fastening. The first and second sections may be connectable by a screw (i.e., threaded) fastening. The screw fastening may be achieved by screwing together complementary threaded ends of the first and second sections. For example, one of the first and second sections may have an external thread and the other of the first and second sections may have a complementary internal thread.

The menstrual hygiene device may further comprise a covering for the thumb and/or a finger (e.g., forefinger and/or middle finger). The covering may be a protective covering. The covering may be for the thumb and forefinger and/or middle finger of the same hand. The covering may be a tightly fitting protective cover such as a sheath. The covering may be for hygiene purposes. The covering may cover at least part of the thumb and the finger (e.g., forefinger and/or middle finger). The covering may cover at least the top portion of the thumb and the finger (e.g., forefinger and/or middle finger). The covering may cover the top half of the thumb and the finger (e.g., forefinger and/or middle finger). The covering may consist of a single component, i.e., one cover, that covers the thumb and the finger (e.g., forefinger and/or middle finger). The single component may be a single, integrally formed component. Alternatively, the covering may comprise two or more components i.e., two or more covers, that cover the thumb and the finger (e.g., forefinger and/or middle finger), for example, the covering may consist of two components i.e., two covers, that cover the thumb and the finger (e.g., forefinger and/or middle finger). The two or more components i.e., two or more covers, may be connected to one another. The two or more components i.e., two or more covers, may not be connected to one another (i.e., they may be physically separate components). The covering or each component (e.g., cover) may be frustoconical shaped, tapering from a first, open end to a second, closed end. The covering or each component (e.g., cover) thereof may be thimble shaped. The covering or each component (e.g., cover) may have a protruding rim around the circumference of the open end.

The covering may be made from any suitable material. The covering may be made from a flexible polymeric material. The covering may be made from silicone, for example medical grade silicone. The covering may be made from thermoplastic elastomers (TPE), for example, medical grade thermoplastic elastomers (TPE). The covering may be made from a rigid polymeric material.

The first chamber may be sized to house a menstrual cup. The first chamber may be sized to house at least part of the covering. The first chamber may be sized to house a menstrual cup and at least part of the covering.

The menstrual cup may have any standard shape and dimensions known in the art. The menstrual cup may have a diameter of about 35 mm to about 50 mm. The menstrual cup may have a length of about 50 mm to about 80 mm. The menstrual cup may be sized to hold about 15 mL to about 45 mL fluid, e.g., period blood.

The housing may further comprise a third section which is connectable to the second section. The second and third sections may be connectable by any suitable fastening. For example, the second and third sections may be connectable by a screw (i.e., threaded) fastening, a latch fastening, a snap-fit fastening, or a magnetic snap fastening. The second and third sections may be connectable by a screw (i.e., threaded) fastening.

The second section may comprise an internal member configured for storage of the covering. The internal member may comprise one or more holes or cut outs which are configured to hold the covering in place, within the first chamber. The internal member may comprise two holes or cut outs which are configured to hold the covering in place, within the first chamber. The one or more holes or cut outs may be sized such that the covering or components (e.g., covers) may be placed through the hole or cut out, closed end first, and held on the internal member by the protruding rim on the open end of the covering or each component (e.g., cover), as the protruding rim is sized such that its diameter is greater than that of the one or more holes or cut outs. When the third section is disconnected from the second section, it may provide access to the internal member configured for holding the covering. When the third section is disconnected from the second section, it may provide access to the covering.

When connected, the first and third sections may form a second chamber. Alternatively, the third section may comprise a plate, which forms a second chamber within the third section. When the second chamber is formed within the third section, it may be used for the storage of one or more tablets. The second chamber may be for the storage of sterilising tablets. The second chamber may be for the storage of medicine, for example pain relief medicine. The second chamber may be for the storage of sterilising tablets and medicine, for example, pain relief medicine.

The menstrual hygiene device may further comprise a fourth section connectable to the first section. The first and fourth sections may be connectable by any suitable fastening. For example, the first and fourth sections may be connectable by a screw (i.e., threaded) fastening, a latch fastening, a snap-fit fastening, or a magnetic snap fastening. The first and fourth sections may be connectable by a screw (i.e., threaded) fastening.

The first and fourth sections, when connected, may form a third chamber. The third chamber may be for storing menstrual hygiene products such as sanitary towels. The third chamber may be for storing menstrual hygiene products such as tampons. The third chamber may be for storing paper towel. The third chamber may be for storing one or more tablets such as sterilising tablets and/or medicine, for example, pain relief medicine. The third chamber may be for storing medicine, for example pain relief medicine. The third chamber may comprise a divider. The divider may divide the chamber into two or more sub-chambers. The divider may divide the third chamber into two sub-chambers. The divider may divide the third chamber into three sub-chambers. The divider may be removable, i.e., the divider may be a non-integral part of the chamber and can be removed to provide an undivided chamber.

According to a second aspect, there is provided a method of folding a menstrual cup for insertion into a vagina, the method comprising pressing a rim of the menstrual cup against the post of the device according the first aspect.

The menstrual cup may be folded by a user by pressing the menstrual cup against the post using only the thumb and a finger of the same hand. The menstrual cup may be folded by a user by pressing the menstrual cup against the post using only the thumb and forefinger. The menstrual cup may be folded by a user by pressing the menstrual cup against the post using only the thumb and middle finger.

The device may comprise a stop, as described hereinabove, and the method may comprise pressing the rim of the menstrual cup against the post such that the rim engages with the stop. The stop may inhibit or prevent slippage of the menstrual cup as it is pressed against the post for folding.

The folded menstrual cup may be inserted into a vagina.

The thumb and finger may be covered, prior to the folding of the menstrual cup. The thumb and finger may be covered by any covering described hereinabove.

The method may comprise: separating the first and second sections of the housing to access the menstrual cup, stored within the first chamber; removing the menstrual cup from the device; grasping the menstrual cup between a thumb and a forefinger and/or middle finger; and pressing the menstrual cup against the stop of the post in the first section of the housing of the device to fold the menstrual cup.

The method may comprise: separating the second and third sections of the housing to access the covering held by the internal member of the second section of the housing; removing the covering from the device and placing the covering on the thumb and forefinger and/or middle finger; separating the first and second sections of the housing to access the menstrual cup, stored within the first chamber and removing the menstrual cup from the device; grasping the menstrual cup with thumb and forefinger and/or middle finger, covered by the covering and pressing the menstrual cup against the stop of the post to fold the menstrual cup for insertion into the vagina.

The method may further comprise separating the first and the fourth section of the housing; and accessing the stored items in the fourth section.

The method may further comprise separating the plate from the third section of the housing; and accessing the stored items in the third section.

The menstrual cup may be sterilised before use. The menstrual cup may be sterilised after use. The menstrual cup may be sterilised using a sterilising solution. Sterilisation may comprise combining water and sterilising tablets. The menstrual cup may be sterilised for 30 minutes or less before use. The menstrual cup may be sterilised for 30 minutes or less after use. The menstrual cup may be sterilised for 15 minutes or less before use, for example for 10 minutes or less, or 5 minutes or less. The menstrual cup may be sterilised for 15 minutes or less after use, for example for 10 minutes or less, or 5 minutes or less. The sterilisation may occur within the menstrual hygiene device. For example, the sterilisation may occur in a chamber of the menstrual hygiene device, such as the first or third chamber of the menstrual hygiene device.

According to a third aspect, there is provided a kit of parts for assembling the menstrual hygiene device according to the first aspect. The kit of parts comprises the first and second sections of the housing. The kit of parts may further comprise the third section of the housing. Additionally, the kit of parts may further comprise the fourth section of the housing. The kit of parts may further comprise the covering. The kit of parts may further comprise a menstrual cup.

The skilled person will appreciate that, except where mutually exclusive, a feature described in relation to any one of the above aspects may be applied mutatis mutandis to any other aspect. Furthermore, except where mutually exclusive, any feature described herein may be applied to any aspect and/or combined with any other feature described herein. Figures

Embodiments will now be described by way of example only, with reference to the Figures, in which:
Figure 1 is a perspective view of a menstrual hygiene device;
Figure 2 is an exploded, side view of the menstrual hygiene device of Figure 1;
Figure 3 is an exploded, cross-sectional view of the menstrual hygiene device of Figure 1 ;
Figure 4 is a cross-sectional view of the menstrual hygiene device of Figure 1;
Figure 5 is an exploded, perspective view of the menstrual hygiene device of Figure 1 ;
Figure 6 is an exploded, perspective view of the menstrual hygiene device of Figure 1 illustrating the positioning of a menstrual cup and covers for the thumb and finger;
Figure 7 is a perspective view of an alternative menstrual hygiene device;
Figure 8 is a cross-sectional view of the menstrual hygiene device of Figure 7, illustrating the position of the menstrual cup and the covering in the device;
Figure 9 is an exploded, perspective view of the menstrual hygiene device of Figure 7, illustrating the position of the menstrual cup and the covering in the device;
Figure 10 is a cross-sectional view of a selection of posts having varying shapes and dimensions;
Figure 11 is a cross-sectional view of another selection of posts with varying shapes and dimensions; Figure 12 shows schematically a menstrual cup being pressed against the post of a menstrual hygiene device; and
Figure 13 shows schematically the menstrual cup of Figure 12 being folded against the post.

### Detailed description

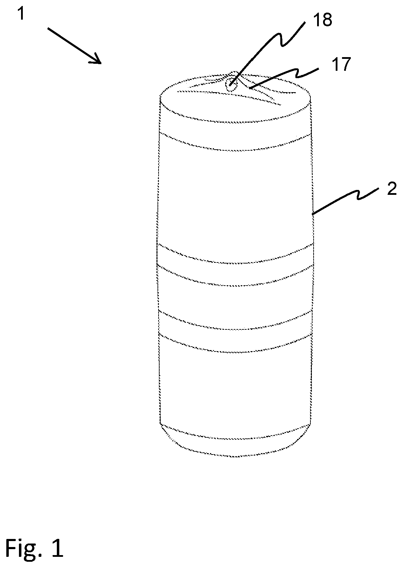

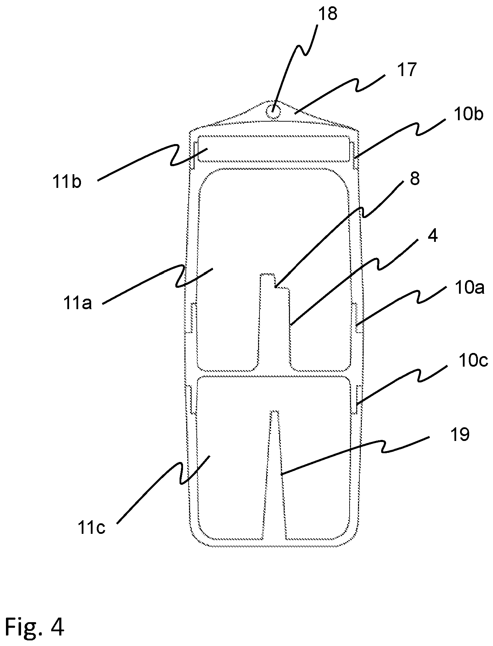

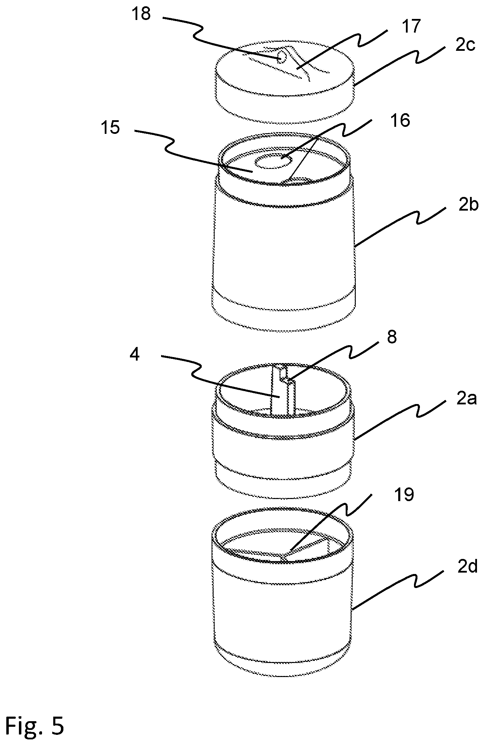

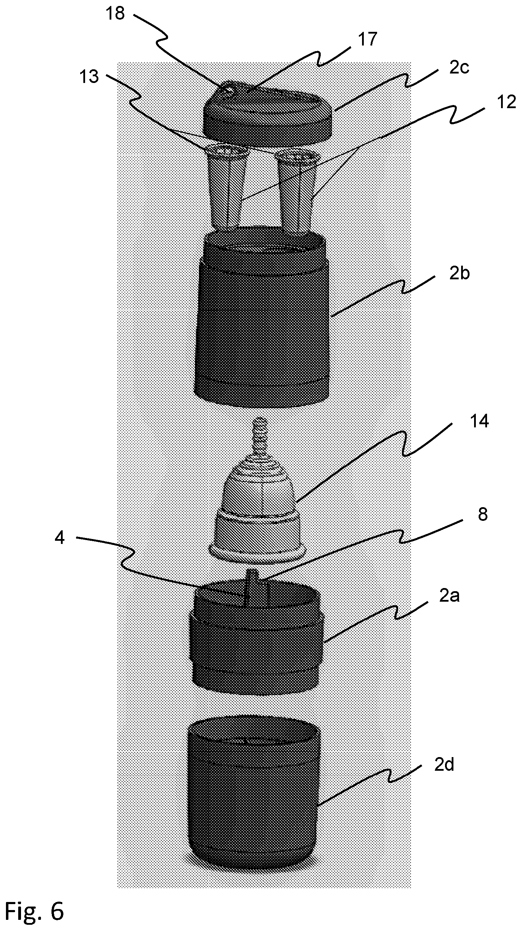

With reference to Figures 1 to 6, a menstrual hygiene device 1 comprises a compact, generally cylindrical housing 2. The housing 2 comprises a first section 2a, second section 2b, third section 2c and fourth section 2d. The housing is made from the rigid material acetonitrile butadiene styrene (ABS).

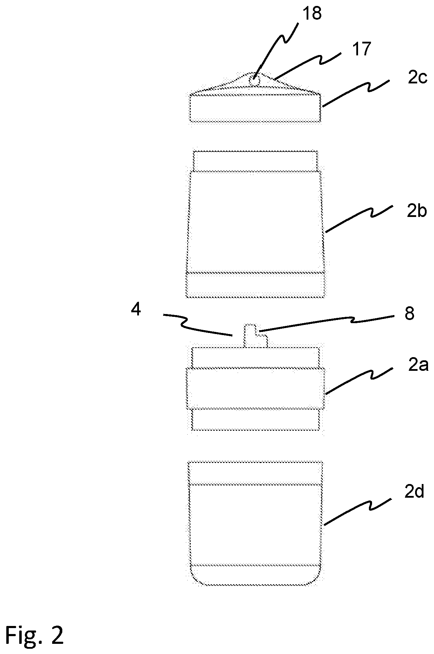

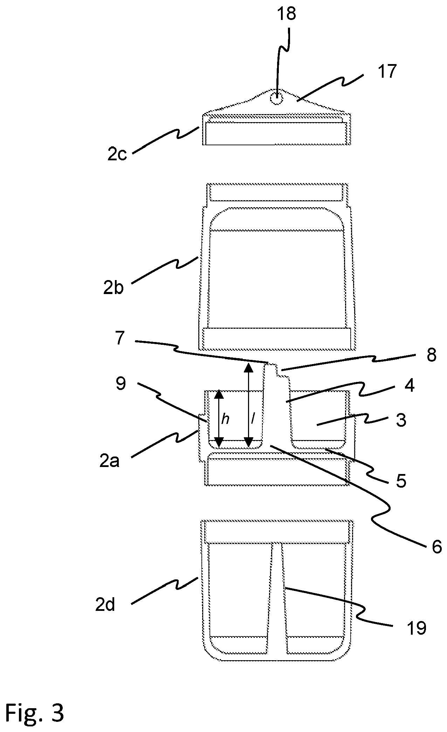

The first section 2a has a well 3 which comprises a post 4. The post is made from the same material as the housing and is integrally formed with the housing. The post is positioned centrally within the well and extends substantially orthogonal to a base of the well 5. The post extends between a first end 6 at a point of attachment to the base of the well and a second end 7 distal from the point of attachment. The post is solid, i.e. , is not hollow or does not comprise a channel. In this embodiment, the post is generally slat shaped. At the second end, the post has a cut-out portion which forms a ledge 8 known as a stop. The cut-out portion is a 90° cut out of the post. The well has a side wall 9 having a height h and the post has a length I greater than that height such that the post and the stop protrudes out of the well beyond the side wall of the first housing section (see, for example, Figures 2 and 3).

The first section of the housing 2a is connectable to the second section of the housing 2b, by a screw fastening 10a. In this embodiment, the first section of the housing has an externally threaded portion (not shown) and the second section of the housing has a corresponding internally threaded portion (not shown) which are configured to retain the first and second sections together when mated. When connected, the first and second sections of the housing form a chamber 11a.

The menstrual hygiene device further comprises a covering 12 for the thumb and forefinger or middle finger. The covering is for hygiene purposes. The covering is made from medical grade silicone. The covering comprises two covers, a cover for the thumb and a cover for the forefinger or middle finger, which cover the top half of the thumb and forefinger or middle finger and are thimble shaped. In this embodiment, the covers of the covering are not connected to one another. The covers taper from a first, open end to a second, closed end. The covers have a protruding rim 13 around the circumference of the open end. The chamber 11a is sized to house a menstrual cup 14 and at least part of the covering 12.

The second section of the housing 2b comprises an internal member 15 which is configured for holding the covering 12. The internal member 15 comprises two holes 16. The two holes 16 are sized such that a cover 12 may be placed through a hole 16, closed end first, and retained by the internal member 15 by the protruding rim 13 on the open end of the cover, the open end of the cover 12 with the protruding rim 13 being sized such that its diameter is greater than that of the two holes.

The second section of the housing 2b is connectable to the third section of the housing 2c, by a screw fastening 10b. In this embodiment, the second section of the housing has an internally threaded portion (not shown) and the third section of the housing has a corresponding externally threaded portion (not shown) which are configured to retain the second and third sections together when mated. When connected, the second and third sections of the housing form a chamber 11b. When the third section 2c is disconnected from the second section 2b it provides access to the internal member 15 of the second section of the housing 2b and as such, access to the covering 12.

The third section of the housing 2c comprises a ridge 17 with an aperture 18. The ridge 17 and aperture 18 are configured to enable attachment of the device to a garment, bag or the like by known attachment means (e.g., a hook, a carabiner, or a rope).

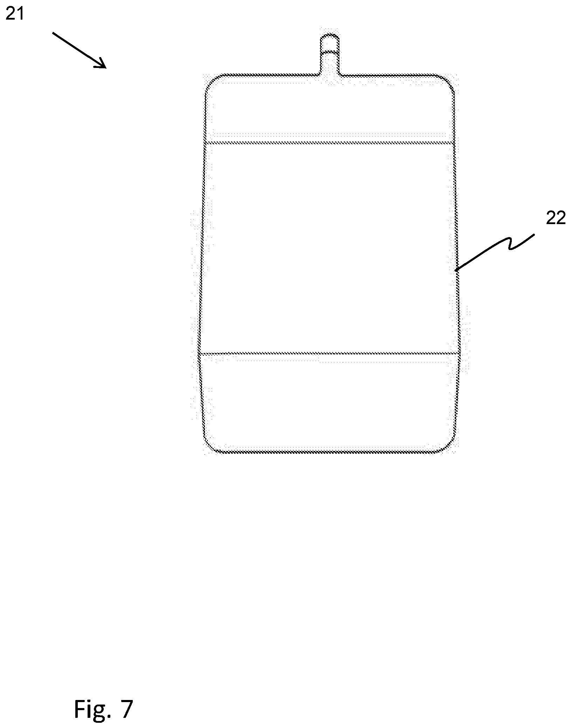

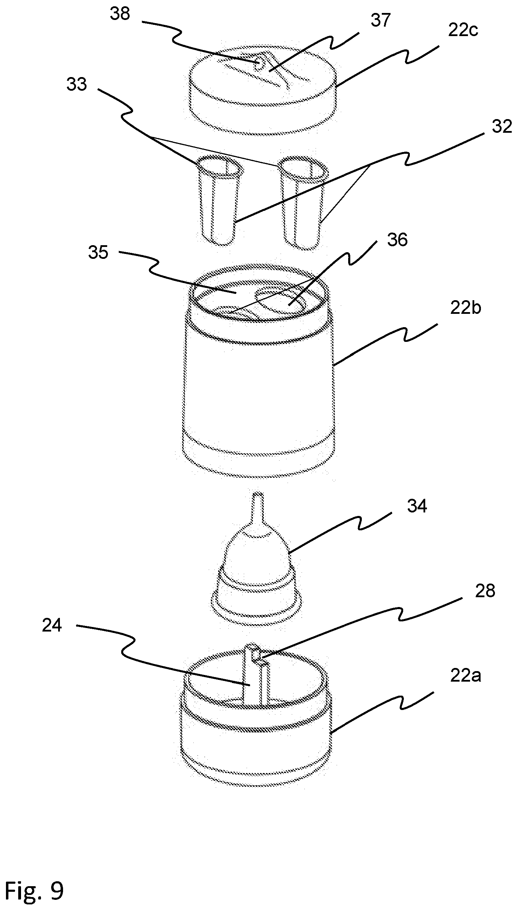

The first section of the housing 2a is connectable to the fourth section of the housing 2d, by screw fastening 10c. In this embodiment, the first section of the housing has an internally threaded portion (not shown) and the fourth section of the housing has a corresponding externally threaded portion (not shown) which are configured to retain the first and fourth sections together when mated. When connected, the first and fourth section of the housing form a chamber 11c. The chamber 11c is suitable for storing menstrual hygiene products such as sanitary towels and/or tampons and/or storing paper towel. The chamber 11c is also suitable for storing one or more tablets, such as sterilising tablets and/or medicine, for example, pain relief medicine. The chamber 11c is suitable for storing medicine, for example pain relief medicine. In this embodiment, the chamber 11c comprises a divider 19 that divides the chamber into three subchambers. With reference to Figures 7 to 9, a further menstrual hygiene device 21 is shown comprising a compact, generally cylindrical housing 22. The device is a smaller version of device described above, differing primarily in that it does not contain a fourth section. The housing 22 comprises a first section 22a, second section 22b and third section 22c. The housing is made from the rigid material acetonitrile butadiene styrene (ABS).

Like the larger version of the device, the first section 22a has a well which comprises a post 24. The post is made from the same material as the housing and is integrally formed with the housing. The post is positioned centrally within the well and extends substantially orthogonal to a base of the well. The post extends between a first end at a point of attachment to the base of the well and a second end distal from the point of attachment. The post is solid, i.e. , is not hollow or does not comprise a channel. In this embodiment, the post is generally slat shaped. At the second end, the post has a cutout portion which forms a ledge 28 known as a stop. The cut-out portion is a 90° cut out of the post. The well has a side wall having a height and the post has a length greater than that height such that the post and the stop protrudes out of the well beyond the side wall of the first housing section (see, for example, section 2a of Figures 2 and 3).

The first section of the housing 22a is connectable to the second section of the housing 22b, by a screw fastening 30a. In this embodiment, the first section of the housing has an externally threaded portion (not shown) and the second section of the housing has a corresponding internally threaded portion (not shown) which are configured to retain the first and second sections together when mated. When connected, the first and second sections of the housing form a chamber 31a.

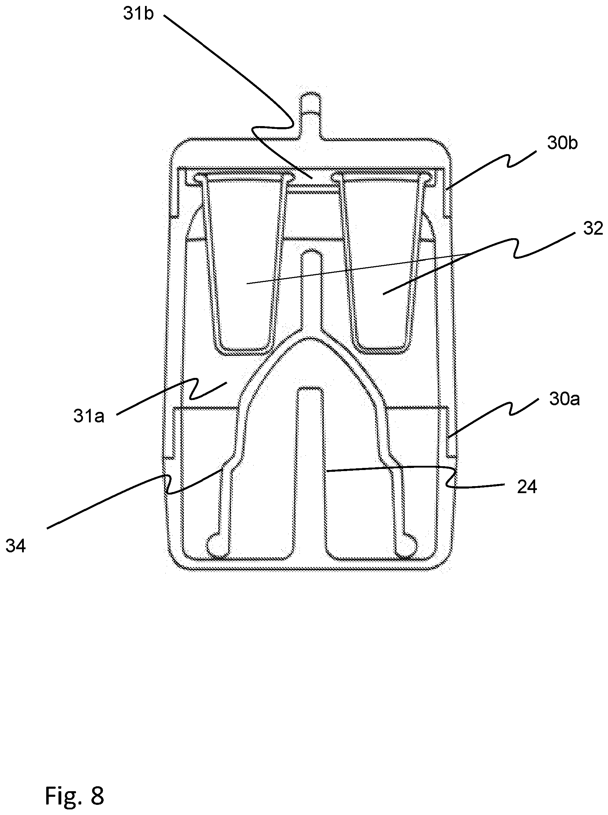

The menstrual hygiene device further comprises a covering 32 for the thumb and forefinger or middle finger. The covering is for hygiene purposes. The covering is made from medical grade silicone. The covering comprises two covers, a cover for the thumb and a cover for the forefinger or middle finger, which cover the top half of the thumb and forefinger or middle finger and are thimble shaped. In this embodiment, the covers of the covering are not connected to one another. The covers taper from a first, open end to a second, closed end. The covers have a protruding rim 33 around the circumference of the open end. The chamber is sized to house a menstrual cup 34 and at least part of the covering 32 (see, for example, Figure 8).

The second section of the housing 22b comprises an internal member 35 which is configured for holding the covering 32. The internal member 35 comprises two holes 36. The two holes 36 are sized such that a cover 32 may be placed through a hole 36, closed end first, and retained by the internal member 35 by the protruding rim 33 on the open end of the cover, the open end of the cover 32 with the protruding rim 33 being sized such that its diameter is greater than that of the two holes.

The second section of the housing 22b is connectable to the third section of the housing 22c, by a screw fastening 30b. In this embodiment, the second section of the housing has an internally threaded portion (not shown) and the third section of the housing has a corresponding externally threaded portion (not shown) which are configured to retain the second and third sections together when mated. When connected, the second and third sections of the housing form a chamber 31 b. When the third section 22c is disconnected from the second section 22b it provides access to the internal member 35 of the second section of the housing 22b and as such, access to the covering 32.

The third section of the housing 22c comprises a ridge 37 with an aperture 38. The ridge 37 and aperture 38 are configured to enable attachment of the device to a garment, bag or the like by known attachment means (e.g., a hook, a carabiner, or a rope).

Use of menstrual hygiene devices 1 and 21 will now be described.

When not in use, the menstrual cup 14 or 34 may be stored in chamber 11a or 31a. When not in use, the covering 12 or 32 is placed in the two holes 16 or 36 of internal member 15 or 35 and held on the internal member by the protruding ridge 13 or 33 of the covering.

When a user wishes to use the menstrual hygiene device 1 or 21 , the second 2b or 22b and third 2c or 22c sections of the housing are separated by unscrewing, to access the covering 12 or 32 held by the internal member 15 or 35 of the second section. The covering is removed from the device and one cover is placed on the thumb and the other cover is placed on either the forefinger or the middle finger. The first 2a or 22a and second 2b or 22b sections of the housing are separated by unscrewing, to access the menstrual cup 14 or 34 held within the chamber formed by the first and second sections 11a or 31a. The menstrual cup is grasped and removed from the device using the covered thumb and forefinger or middle finger. The menstrual cup is then pressed against the stop 8 or 28 of the post 4 or 24, to fold the menstrual cup, for insertion into the vagina. The menstrual cup is then inserted into the vagina.

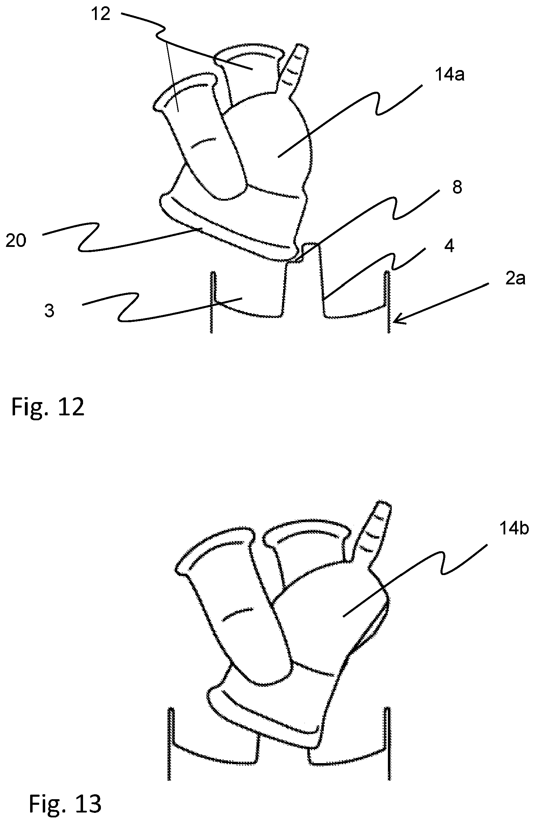

More particularly, with reference to Figures 12 and 13, which are applicable to the device of Figures 1 to 6 and the device of Figures 7 to 9, the rim 20 of the menstrual cup 14a is placed against the post 4 positioned within the well 3 of the first section of the housing 2a. The rim 20 of the menstrual cup 14a engages with the stop 8 of the post 4. When the menstrual cup 14a is pressed against the stop 8 of the post 4 by a user, this inhibits slippage of the menstrual cup down the post. Because the menstrual cup is made of a resilient material such as silicone, the menstrual cup folds (as illustrated in Figure 13) as the user continues to press the cup against the post, to provide a folded menstrual cup 14b. The menstrual cup is folded using only the thumb and forefinger or middle finger, which are covered. The covering 12, made from medical grade silicone and provided for hygiene purposes, comprises two covers, a cover for the thumb and a cover for the forefinger or middle finger, which cover the top half of the thumb and forefinger or middle finger and are thimble shaped.

Further variations and modifications may be made within the scope of the invention herein disclosed.

In particular, it will be appreciated that the shape of the housing can be adapted. For example, the housing could be square, rectangular, triangular, or hexagonal in cross section perpendicular to the longitudinal axis.

The housing may be made from any suitable material. For example, the housing may be made from any suitable rigid polymeric material. The housing may be made from a polyethylene, such as high-density polyethylene (HDPE).

The housing could comprise a first, second and third section only, for example, when the device is to be made more compact and therefore further storage provided by a fourth section of the housing is not required. The housing may be provided with any suitable means (e.g., any suitable connector) for attaching the device to a garment, bag or the like by known attachment means, for example a hook, a carabiner, or a rope. The housing may not comprise means (e.g., any suitable connector) for attaching the device to a garment, bag or the like, such as a ridge with an aperture.

The shape and size of the post could be varied. For example, the post could be column shaped, pillar shaped, cuboidal shaped, cylindrical shaped or triangular prism shaped.

The side walls of the post could be substantially parallel to one another and orthogonal to the base of the well. One or both of the side walls of the post could be sloping, e.g., one of the side walls of the post could be sloping. For example, one or both of the side walls could be sloping such that the side wall meets the base of the well at an acute angle (an angle less than 90°) or one or both of the side walls may be sloping such that the side wall meets the base of the well at an obtuse angle (an angle greater than 90°, but less than 180°).

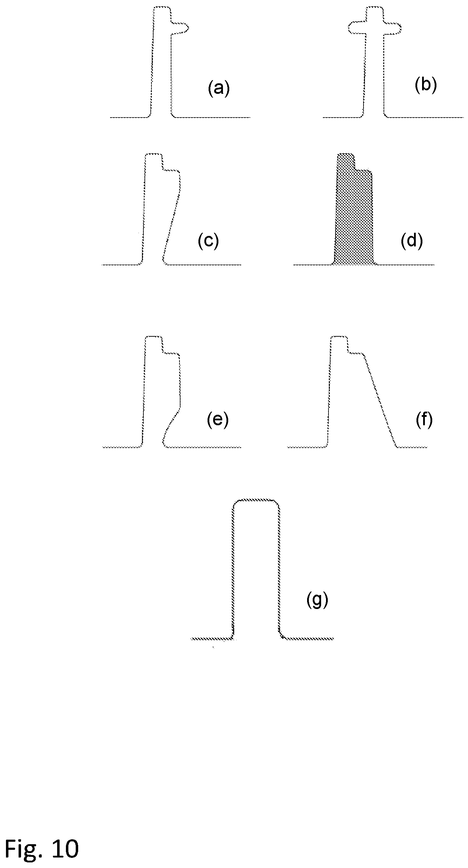

One or both of the side walls of the posts may slope from the first end of the post to the second end of the post, e.g., one of the side walls of the posts may slope from the first end of the post to the second end of the post. For example, the post of figure 10(f) has one side wall which is orthogonal to the base of the well and one side wall that slopes from the ledge at the second end of the post, distal to the base of the well to the first end of the post at the point of attachment to the base of the well, such that the sloping side wall meets the base of the well an obtuse angle.

One or both side wall of the post may comprise a parallel section and a sloping section across their length. For example, one or both of the side wall could have a sloping section from the first end of the post and a parallel section from second end of the post.

The sloping section may be half of the length of the post, e.g., from the second end of the post.

The sloping section may be a third of the length of the post, e.g., from the second end of the post. For example, the post of Figure 10(e) has one side wall which is orthogonal to the base of the well; and one side wall comprises a parallel section, which is about two thirds of the side wall and extends from the ledge at the second end of the post, distal to the base of the well; and a sloping section, which is about one third of the side wall and extends from the end of the parallel section to the first end of the post at the point of attachment on the base of the well, such that the sloping section of the side wall meets the base of the well at an acute angle.

The sloping section may be two thirds of the length of the post e.g., from the second end of the post. For example, the post of Figure 10(d) has one side wall which is orthogonal to the base of the well; and one side wall comprises a parallel section, which is about one third of the side wall and extends from the ledge at the second end of the post, distal to the base of the well; and a sloping section, which is about two thirds of the side wall and extends from the end of the parallel section to the first end of the post at the point of attachment on the base of the well, such that the sloping section of the side wall meets the base of the well at an acute angle.

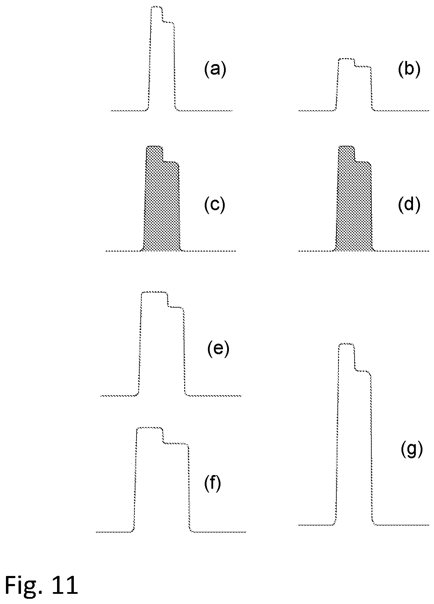

The post can have any suitable dimensions, i.e., any suitable length and width. The post could have a length of from about 20 mm to about 50 mm, for example from about 30 to about 40 mm. The post may have a length of about 30 mm to about 60 mm, for example from about 40 mm to about 50 mm. The length of the post refers to the longest longitudinal axis of the post extending orthogonally from the base of the well to the distal end of the post. Figure 11(b), (d), and (g) show posts with varying lengths. The post may have a width of from about 5 mm to about 40 mm, for example from about 10 mm to about 30 mm, or from about 10 mm to about 20 mm, or from about 10 mm to about 15 mm. The width of the post refers to the longest axis of the post, orthogonal to the longest longitudinal axis, and disregarding any stop portion or section. Figure 11 (a), (c), (e), and (f) show posts with varying widths.

The post may not have a stop (see, for example, Figure 10(g)). Alternatively, a stop may be present and the shape and size of the stop could also be varied. For example, the stop could be formed by a cut-out portion of the post, the stop could be formed by a ledge that protrudes orthogonally from a point on the body of the post, the stop could be formed by a ledge that protrudes orthogonally around the circumference of the post. The stop could be at the second end of the post or could be close to the second end of the post. It could be that the stop (e.g., the ledge) is formed by a protrusion. The protrusion may be located on one side of the post or on two or more sides of post. For example, the post of Figure 10(a) has two side walls which are substantially parallel to one another and orthogonal to the base of the well. One side wall comprises a protrusion, proximate to the second end of the post.

The protrusion may extend around the entire post, i.e., around the circumference of the post. For example, the post of Figure 10(b) has two side walls which are substantially parallel to one another and orthogonal to the base of the well. The post comprises a protrusion that extends around the circumference of the post, such that it provides a protruding ridge, proximate to the second end of the post.

For the avoidance of doubt, highlighted posts (Figure 10(d), Figure 11 (c) and Figure 11(d)) is the post of embodiments shown in Figures 2-6, 8, 9 and Figures 12 and 13.

The post could have a length equal to or less than the height of the side wall of the well. It could be that the stop is positioned on the post such that it is within boundary of the well.

The sections of the housing are connectable by any suitable fastening. For example, the sections of the housing could be fastened to one another by a latch fastening, a snap-fit fastening, or a magnetic fastening.

The covering may be any suitable apparatus for covering a thumb and a forefinger and/ or a middle finger for hygiene purposes. The covering may be a single component, i.e., single cover, capable of covering a thumb and a forefinger and/or middle finger. The covering could be two or more components, for example, three components (i.e., three covers) that cover a thumb, forefinger and middle finger. Where the covering could be two or more components, i.e., two or more covers, said components (i.e., covers) may be connected to one another. The covering could cover substantially all of the thumb and forefinger and/ or middle finger.

The covering may be made from any suitable material. For example, the covering may be made from a flexible or rigid polymeric material. The covering may be made from a thermoplastic elastomer (TPE), such as medical grade thermoplastic elastomer (TPE). The internal member of the second housing could be any suitable size or shape so long as it is configured to retain the covering in place.

The third section of the housing could comprise a plate, forming a chamber within the third section of the housing, which may be suitable for the storage of one or more tablets, such as sterilising tablets and/ or medicine (e.g., pain relief medicine).

The chamber formed by the connecting the first and fourth sections of the housing may not comprise a divider, for example the chamber could be undivided. When a divider is present, it could divide the chamber into any number of required sub-chamber, for example two, three or four sub-chambers. The divider may be removable, i.e., the divider may be a non-integral part of the chamber and can be removed to provide an undivided chamber.

It will be understood that the invention is not limited to the embodiments described above and various modifications and improvements can be made without departing from the concepts described herein. Except where mutually exclusive, any of the features may be employed separately or in combination with any other features and the disclosure extends to and includes all combinations and sub-combinations of one or more features described herein, within the scope of the claims.

## Claims

1. A menstrual hygiene device (1, 21) comprising a housing (2, 22) and a well (3) within the housing, the well comprising a post (4, 24) against which a menstrual cup (14, 34; 14a, 14b) is foldable using only the thumb and a finger of the same hand, **characterized in that** the post (14, 24) is solid.

2. The menstrual hygiene device (1, 21) according to claim 1, wherein the post (4, 24) has a stop (8, 28) for retaining the menstrual cup (14, 34; 14a, 14b) in position against the post (4, 24) during folding, for example, wherein the stop (8, 28) is formed by a cut-out portion, ledge or notch of the post (4, 24).

3. The menstrual hygiene device (1, 21) according to any preceding claim, wherein the post (4, 24) is generally slat shaped.

4. The menstrual hygiene device (1, 21) according to any preceding claim, wherein the housing (2, 22) comprises a first section (2a, 22a) and a second section (2b, 22b) which are connectable to each other, wherein the well (3) is comprised within the first section (2a, 22a), and wherein, when connected, the first (2a, 22a) and second (2b, 22b) sections form a first chamber (11a, 31a).

5. The menstrual hygiene device (1, 21) according to any preceding claim, further comprising a covering (12, 32) for the thumb and/or forefinger.

6. The menstrual hygiene device (1, 21) according to claims 4 and 5, wherein the first chamber (11a, 31a) is sized to house a menstrual cup (14, 34; 14a, 14b), wherein the first chamber (11a, 31a) is sized to house at least part of the covering (12, 32), optionally wherein the second section (2b, 22b) comprises an internal member (15, 35) configured to retain the covering (12, 32).

7. The menstrual hygiene device (1, 21) according to any of claims 4 to 6, wherein the housing (2, 22) further comprises a third section (2c, 22c) which is connectable to the second section (2b, 22b), and which provides access to the covering (12, 32) when disconnected from the second section (2b, 22b).

8. The menstrual hygiene device (1, 21) according to claim 7, wherein the third section (2c, 22c) comprises a second chamber (11b, 31b), for example, for the storage of one or more tablets, such as sterilising tablets and/or medicine.

9. The menstrual hygiene device (1, 21) according to any of claims 4 to 8, further comprising a fourth section (2d) connectable to the first section (2a, 22a), and wherein when connected, the first and fourth sections form a third chamber (11c), for example, for storing menstrual hygiene products such as tampons.

10. A method of folding a menstrual cup (14, 34; 14a, 14b) for insertion into a vagina, the method comprising pressing a rim (20) of the menstrual cup (14, 34; 14a, 14b) against the post (4, 24) of the device (1, 21) according to any of claims 1 to 9.

11. The method according to claim 10, wherein the menstrual cup (14a, 14b) is folded by a user by pressing the menstrual cup (14, 34; 14a, 14b) against the post (4, 24) using only the thumb and a finger of the same hand, for example, the thumb and forefinger, or thumb and middle finger.

12. The method according to claim 10 or 11, wherein the device (1, 21) comprises the stop (8, 28) according to claim 2 and the rim (20) of the menstrual cup (14, 34; 14a, 14b) engages with the stop (8, 28).

13. The method according to claim 11 or 12, wherein the thumb and finger are covered, prior to the folding of the menstrual cup (14, 34; 14a, 14b).

14. The method of according to any one of claims 10 to 13, wherein the menstrual cup (14, 34; 14a, 14b) is sterilised before or after use.

15. A kit of parts for use in a method according to any one of claims 10 to 14, comprising the device (1, 21) of any one of claims 1 to 9 and a menstrual cup (14, 34; 14a, 14b).

## Patentansprüche

1. Eine Menstruationshygienevorrichtung (1, 21), die ein Gehäuse (2, 22) und eine Vertiefung (3) aufweist, wobei die Vertiefung einen Stift (4, 24) aufweist, an dem eine Menstruationstasse (14, 34; 14a, 14b) allein mit dem Daumen und einem Finger derselben Hand zusammengefaltet werden kann, **dadurch gekennzeichnet, dass** der Stift (14, 24) massiv ist.

2. Die Menstruationshygienevorrichtung (1, 21) nach Anspruch 1, wobei der Stift (4, 24) einen Anschlag (8, 28) aufweist, um die Menstruationstasse (14, 34; 14a, 14b) während des Zusammenfaltens in Position gegen den Stift (4, 24) zu halten, wobei der Anschlag (8, 28) beispielsweise durch einen ausgeschnittenen Abschnitt, eine Leiste oder eine Kerbe des Stifts (4, 24) ausgebildet wird.

3. Die Menstruationshygienevorrichtung (1, 21) nach einem der vorhergehenden Ansprüche, wobei der Stift (4, 24) im Wesentlichen lamellenförmig ist.

4. Die Menstruationshygienevorrichtung (1, 21) nach einem der vorhergehenden Ansprüche, wobei das Gehäuse (2, 22) einen ersten Abschnitt (2a, 22a) und einen zweiten Abschnitt (2b, 22b) aufweist, die miteinander verbindbar sind, wobei die Vertiefung (3) innerhalb des ersten Abschnitts (2a, 22a) angeordnet ist und wobei im verbundenen Zustand der erste (2a, 22a) und der zweite (2b, 22b) Abschnitt im verbundenen Zustand eine erste Kammer (11a, 31a) ausbilden.

5. Die Menstruationshygienevorrichtung (1, 21) nach einem der vorhergehenden Ansprüche, die ferner eine Abdeckung (12, 32) für den Daumen und/oder den Zeigefinger aufweist.

6. Die Menstruationshygienevorrichtung (1, 21) nach den Ansprüchen 4 und 5, wobei die erste Kammer (11a, 31a) zur Aufnahme einer Menstruationstasse (14, 34; 14a, 14b) dimensioniert ist, wobei die erste Kammer (11a, 31a) so dimensioniert ist, dass sie zumindest einen Teil der Abdeckung (12, 32) aufnehmen kann, wobei der zweite Abschnitt (2b, 22b) optional ein inneres Element (15, 35) aufweist, das so ausgebildet ist, dass es die Abdeckung (12, 32) hält.

7. Die Menstruationshygienevorrichtung (1, 21) gemäß einem der Ansprüche 4 bis 6, wobei das Gehäuse (2, 22) außerdem einen dritten Abschnitt aufweist (2c, 22c), der mit dem zweiten Abschnitt (2b, 22b) verbindbar ist und der, wenn er vom zweiten Abschnitt (2b, 22b) getrennt ist, Zugang zur Abdeckung (12, 32) gewährt.

8. Die Menstruationshygienevorrichtung (1, 21) nach Anspruch 7, wobei der dritte Abschnitt (2c, 22c) eine zweite Kammer (11b, 31b) aufweist, beispielsweise zur Aufbewahrung einer oder mehrerer Tabletten, wie Desinfektionstabletten und/oder Medikamente.

9. Die Menstruationshygienevorrichtung (1, 21) nach einem der Ansprüche 4 bis 8, die außerdem einen vierten Abschnitt (2d) aufweist, der mit dem ersten Abschnitt (2a, 22a) verbindbar ist, und wobei der erste und der vierte Abschnitt, wenn verbunden, eine dritte Kammer (11c) ausbilden, beispielsweise zur Aufnahme von Menstruationshygieneprodukten wie Tampons.

10. Verfahren zum Zusammenfalten einer Menstruationstasse (14, 34; 14a, 14b) zum Einführen in eine Vagina, wobei das Verfahren ein Andrücken eines Randes (20) der Menstruationstasse (14, 34; 14a, 14b) gegen den Stift (4, 24) der Vorrichtung (1, 21) gemäß einem der Ansprüche 1 bis 9 aufweist.

11. Verfahren nach Anspruch 10, wobei die Menstruationstasse (14a, 14b) von einem Benutzer gefaltet wird, indem die Menstruationstasse (14, 34; 14a, 14b) nur mit dem Daumen und einem Finger derselben Hand gegen den Stift (4, 24) gedrückt wird, beispielsweise mit dem Daumen und dem Zeigefinger oder dem Daumen und dem Mittelfinger.

12. Verfahren nach Anspruch 10 oder 11, wobei die Vorrichtung (1, 21) den Anschlag (8, 28) nach Anspruch 2 aufweist und der Rand (20) der Menstruationstasse (14, 34; 14a, 14b) in den Anschlag (8, 28) eingreift.

13. Verfahren nach Anspruch 11 oder 12, wobei der Daumen und der Finger vor dem Zusammenfalten der Menstruationstasse (14, 34; 14a, 14b) mit einer Abdeckung bedeckt werden.

14. Verfahren nach einem der Ansprüche 10 bis 13, wobei die Menstruationstasse (14, 34; 14a, 14b) vor oder nach der Verwendung sterilisiert wird.

15. Ein Teilesatz zur Verwendung in einem Verfahren gemäß einem der Ansprüche 10 bis 14, der eine Vorrichtung (1, 21) gemäß einem der Ansprüche 1 bis 9 aufweist und eine Menstruationstasse (14, 34; 14a, 14b).

## Revendications

1. Un dispositif d'hygiène menstruelle (1, 21) comprenant un boîtier (2, 22) et un puits (3) situé à l'intérieur du boîtier, le puits comprenant un montant (4, 24) contre lequel une coupe menstruelle (14, 34 ; 14a, 14b) peut être pliée uniquement à l'aide du pouce et d'un doigt de la même main, **caractérisé en ce que** le montant (4, 24) est rigide.

2. Le dispositif d'hygiène menstruelle (1, 21) selon la revendication 1, dans lequel le montant (4, 24) comporte une butée (8, 28) destinée à maintenir la coupe menstruelle (14, 34 ; 14a, 14b) en position contre le montant (4, 24) pendant le pliage, la butée (8, 28) étant, par exemple, formée par une partie découpée, un rebord ou une encoche du montant (4, 24).

3. Le dispositif d'hygiène menstruelle (1, 21) selon l'une quelconque des revendications précédentes, dans lequel le montant (4, 24) a globalement la forme d'une latte.

4. Le dispositif d'hygiène menstruelle (1, 21) selon l'une quelconque des revendications précédentes, dans lequel le boîtier (2, 22) comprend une première section (2a, 22a) et une deuxième section (2b, 22b) qui peuvent être assemblées l'une à l'autre, dans lequel le puits (3) est compris à l'intérieur de la première section (2a, 22a), et dans lequel, lorsqu'elles sont assemblées, la première (2a, 22a) et la deuxième (2b, 22b) section forment une première chambre (11a, 31a).

5. Le dispositif d'hygiène menstruelle (1, 21) selon l'une quelconque des revendications précédentes, comprenant en outre une protection (12, 32) destinée à recouvrir le pouce et/ou l'index.

6. Le dispositif d'hygiène menstruelle (1, 21) selon les revendications 4 et 5, dans lequel la première chambre (11a, 31a) est dimensionnée pour loger une coupe menstruelle (14, 34 ; 14a, 14b) et au moins une partie de la protection (12, 32), et dans lequel, éventuellement, la deuxième section (2b, 22b) comprend un élément interne (15, 35) conçu pour retenir la protection (12, 32).

7. Le dispositif d'hygiène menstruelle (1, 21) selon l'une quelconque des revendications 4 à 6, dans lequel le boîtier (2, 22) comprend en outre une troisième section (2c, 22c) pouvant être assemblée à la deuxième section (2b, 22b) et permettant d'accéder à la protection (12, 32) lorsqu'elle est désolidarisée de la deuxième section (2b, 22b).

8. Le dispositif d'hygiène menstruelle (1, 21) selon la revendication 7, dans lequel la troisième section (2c, 22c) comprend une deuxième chambre (11b, 31b), destinée, par exemple, au stockage d'un ou de plusieurs comprimés, tels que des comprimés de stérilisation et/ou des médicaments.

9. Le dispositif d'hygiène menstruelle (1, 21) selon l'une quelconque des revendications 4 à 8, comprenant en outre une quatrième section (2d) pouvant être assemblée à la première section (2a, 22a), les première et quatrième sections formant, lorsqu'elles sont assemblées, une troisième chambre (11c), destinée, par exemple, au stockage de produits d'hygiène menstruelle tels que des tampons.

10. Une méthode de pliage d'une coupe menstruelle (14, 34 ; 14a, 14b) en vue de son insertion dans un vagin, la méthode comprenant une étape consistant à appuyer un rebord (20) de la coupe menstruelle (14, 34 ; 14a, 14b) contre le montant (4, 24) du dispositif (1, 21) selon l'une quelconque des revendications 1 à 9.

11. La méthode selon la revendication 10, dans laquelle la coupe menstruelle (14a, 14b) est pliée par une utilisatrice en appuyant la coupe menstruelle (14, 34 ; 14a, 14b) contre le montant (4, 24) en utilisant uniquement le pouce et un doigt de la même main, par exemple le pouce et l'index, ou le pouce et le majeur.

12. La méthode selon la revendication 10 ou 11, dans laquelle le dispositif (1, 21) comprend la butée (8, 28) selon la revendication 2 et le rebord (20) de la coupe menstruelle (14, 34 ; 14a, 14b) vient en prise avec la butée (8, 28).

13. La méthode selon la revendication 11 ou 12, dans laquelle le pouce et le doigt sont recouverts avant le pliage de la coupe menstruelle (14, 34 ; 14a, 14b).

14. La méthode selon l'une quelconque des revendications 10 à 13, dans laquelle la coupe menstruelle (14, 34 ; 14a, 14b) est stérilisée avant ou après utilisation.

15. Un ensemble de pièces destiné à être utilisé dans une méthode selon l'une quelconque des revendications 10 à 14, comprenant le dispositif (1, 21) selon l'une quelconque des revendications 1 à 9 et une coupe menstruelle (14, 34 ; 14a, 14b).
